# EUROPEAN PATENT SPECIFICATION

(11) **EP 0 417 330 B1**
(45) Date of publication and mention of the grant of the patent: **17.11.1994**
(21) Application number: 90906436.2
(22) Date of filing: 07.03.1990
(51) Int. Cl.: A61B 17/56, A61F 2/28, A61L 27/00

(54) **DEVICE FOR RESTORING A LOST FRACTION OF TUBULAR BONE**
GERÄT ZUR WIEDERHERSTELLUNG EINES VERLORENEN TEILES EINES RÖHRENKNOCHENS
DISPOSITIF DE RESTAURATION D'UN ELEMENT MANQUANT DANS UN OS TUBULAIRE

(30) Priority: 31.03.1989 SU 4671447
(43) Date of publication of application: 20.03.1991
(73) Proprietor: VSESOJUZNY NAUCHNO-ISSLEDOVATELSKY I ISPYTATELNY INSTITUT MEDITSINSKOI TEKHNIKI, Moscow, 129301 (RU); TSENTRALNY NAUCHNO-ISSLEDOVATELSKY INSTITUT TRAVMATOLOGII I ORTOPEDII IMENI N.N. PRIOROVA, Moscow (RU)
(72) Inventor: DAVYDOV, Anatoly Borisovich, Moscow, 111396 (RU); BELYKH, Sergei Ivanovich, Moscow, 129041 (RU); SHAPOSHNIKOV, July Georgievich, Moscow, 123181 (RU); MASLENNIKOV, Stanislav Grigorievich, Moscow, 123298 (RU); MALAKHOV, Oleg Alexeevich, Moscow, 115551 (RU)
(74) Representative: Lerwill, John
(86) International application number: SU9000065
(87) International publication number: WO9011726

(56) References cited:
- SU-A- 590 872
- SU-A- 601 851
- SU-A- 606 240
- SU-A- 990 193
- SU-A- 1 503 778
- SU-A- 1 503 779

## Description

### Field of the Invention

The invention relates generally to reconstructive traumatology and orthopedics and more specifically to a device for restoration of a tubular bone missing portion.

### Prior Art

One of the problems of bone tissue restoration is to avoid any free space between bone fragments, to make impossible filling of the space occupied earlier by the lost bone, with the connective tissue, and controlled evacuation of said space to leave room for the newly growing tissue.

A prerequisite for solving said problem resides in the development of biocompatible polymers biodegradable under the action of body fluids within preset periods of time, elaboration of technological processes for making diversely shaped finished products from such polymers, as well as of technology for imparting therapeutic properties to such polymer products.

The heretofore-known structures made of biologically indifferent materials are aimed at permanent replacement of a bone missing portion and thus render it completely impossible to form a fresh bone tissue of patient's own at the place involved.

One prior-art device for external immobilization of bone fragments (cf. I.A.Moshkovich, Operative orthopedics, 1983, Meditsina Publishers, Moscow,pp.224-225 (in Russian) is known to comprise a number of rings interconnected through rods and provided with intramedullary pins. Application of said device enables one to elongate the human limb by daily multiply repeated gradual distraction of bone fragments by gradually increasing the distance between the rings. A distraction cycle lasts from 20 to 80 days.

Such a device, however, is suitable for stretching sound limbs only. Besides, its application involves much labour consumption on the part of medical staff within a prolonged period of time, it is a source of permanent danger of infecting at the place of introduction of transverse pins, is causative of contracture of ligaments; its application is absolutely impossible in the case of extensive total bone defects due to a complicated injury or gunshot wounds.

One state-of-the-art device for restoration of a missing portion of a tubular bone (cf. "Biomekhanika", 1975, Riga, V.K.Kalnberz et al. Clinico-biomechanical requirements imposed on the endoprosthesis of the femoral diaphisis",pp.365-366 (in Russian) is known to comprise a central metallic rod and a tubular element based on biologically indifferent composite materials, such as polymers or inorganic substances, said tubular element being fitted on the rod. As to its configuration and dimensions the tubular element corresponds to the bone missing portion. In the course of surgery the rod is introduced into the medullary canals of the tubular bone fragments, whereupon the tubular element is installed on the rod at the place of the bone missing portion and is fixed to the bone fragments. However, such a tubular element is very difficult to be made so as to suit the individual dimensions of patient's injury. It is extremely difficult to combine an artificial bone with the bone matrix; the bone tissue is liable to resorb at the place of contact with the artificial bone and, besides, the reaction to a large foreign body manifests itself continuously after implantation of the artificial bone, and a possibility of its replacement by the genuine bone of the a patient's own is ruled out completely.

### Disclosure of the Invention

It is an object of the present invention to provide a device for restoration of a tubular bone missing portion which would ensure restoration of the limb's function.

The object is accomplished due to the fact that in a device for restoration of a tubular bone missing portion, comprising a central rod insertable into the medullary canals of the bone fragments, and a tubular element made of a synthetic material and fitted on the rod at the place occupied by the lost bone portion, said element being equidimensional with the latter, according to the invention, the tubular element being shaped as a shell of ploymers biocompatible with the bone tissue, and a layer of a polymer porous material introducible between the rod and the shell and incorporating a biomass of homogenized bone tissues.

The proposed device provides for regeneration of the bone tissue on vast areas of the missing bone. This is attained due to an appropriate construction of the tubular element. Provision of the aforesaid shell enables one to prevent the regeneration zone from being filled with soft tissues, makes it possible to retain the porous material with the biomass within the volume of the bone missing portion in the regeneration process, and determines the geometric dimensions of the bone portion being restored. The porous mass plays the part of a matrix, whereon the living bone tissue is being formed. Besides, provision of a layer of the porous mass between the rod and the shell allows of efficiently fixing the tubular element on the rod.

Provision of a biomass of homogenized bone tissues in the porous mass promotes the regeneration process, which leads to a complete restoration of the bone missing portion.

In a preferred embodiment of the present invention the shell is shaped as a telescopic structure made up of at least two tubular elements. This makes it possible to change the shell length immediately during surgery in strict accordance with the length of the bone missing portion, which makes possible the proceeding of the regeneration process and cuts down the operating time.

According to one of the embodiments of the present invention, the layer of a porous polymer material is shaped as a bundle of fibres of biocompatible polymers.

Such an embodiment of the invention rules out the presence of a free space between the rod and the shell, which adds to the reliability of holding the tubular element to the rod and improves the bone tissue regeneration conditions.

According to another embodiment of the invention the layer of a porous polymer material is made of expanded hydrogel.

Such an embodiment likewise makes it possible to fill the entire space between the shell and the rod, facilitates biomass introduction and contributes to its uniform distribution and prolonged dwell in the zone of the bone missing portion.

### Summary of the Drawings

Further objects and advantages of the present invention will become evident from the following detailed description of some specific exemplary embodiments thereof with reference to the accompanying drawings, wherein:
FIG.1 depicts a device, according to the invention, after its having been installed in the limb's tubular bone; and
FIGS 2, 3, 4 represent the various embodiments of the shell construction.

### Preferred Embodiment of the Invention

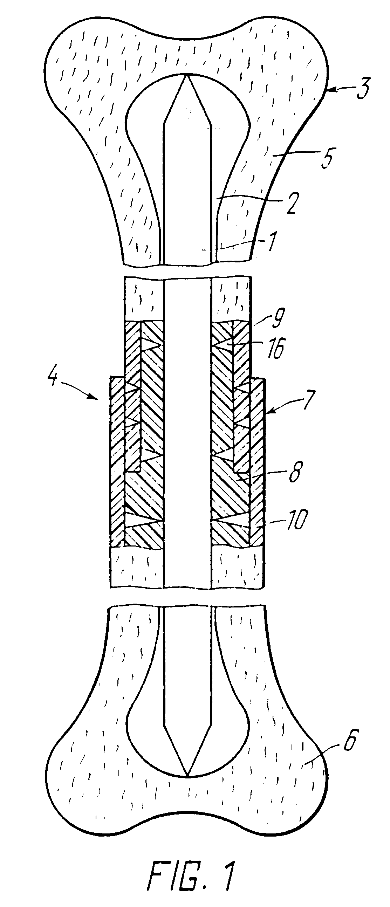

The device of the present invention comprises a central rod 1 (FIG.1) adapted to be introduced into a medullary canal 2 of an injured bone 3, and a tubular element 4 to be fitted on the rod 1 at the place of a bone missing portion 3 so as to contact both of the bone fragments 5, 6 of the bone 3. As to its dimensions the tubular element 4 corresponds to the bone missing portion 3 and is shaped as a shell 7 of the polymers biocompatible with the bone tissue, and a layer 8 of a porous polymer material introducible between the rod 1 and the shell 7 and incorporating a biomass of homogenized bone tissues.

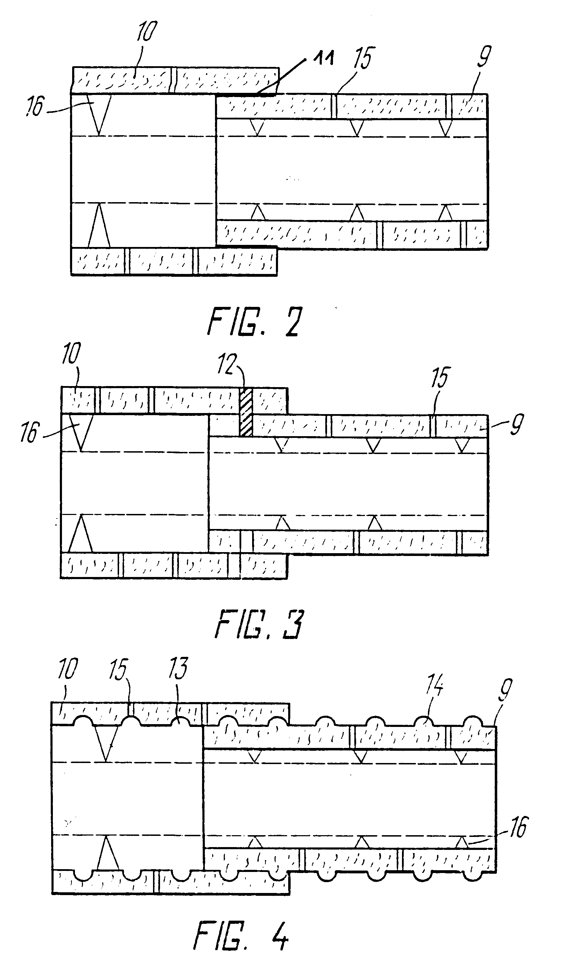

The shell 7 is shaped as a telescopic structure made up of at least two tubular elements 9, 10 (FIGS 2 through 4), which are held to each other. The length of the shell 7 should be equal to the length of the bone missing portion 3. The tubular elements 9, 10 of the shell 7 may be interlinked with the aid of a bonded joint 11 (FIG.2), or a stud 12 (FIG.3), as well as by virtue of a joint consisting of a recess 13 and a ridge 14 (FIG.4).

The shell 7 is provided with radial holes 15, while fixing-and-supporting elements 16 (FIGS 1 through 4) are provided on the inner surface of the tubular elements 9, 10.

The layer 8 of a porous polymer material may be shaped as a bundle of fibres from biocompatible polymers or be made of expanded hydrogel.

The device is fitted onto human as follows.

Prior to installing the device one should select a required set of the device whose standard size depends on the diameter of the central rod 1, which in turn should correspond to the diameter of the medullary canal 2 of the injured bone 3. Upon withdrawing the rod 1 its length is adjusted by cutting so that after having been introduced into the proximal portions of the bone fragments 5, 6 the rod 1 would ensure such a limb length that it has had before injury. Then the coaxial shell 7 is fitted onto the rod 1 approximately in its midportion with the aid of the supporting element 16, whereupon the porous mass 8 is introduced between the rod 1 and the coaxial shell 7 as a bundle of fibres. When the porous mass is used as expanded hydrogel it is introduced at a later stage along with the biomass. Then the rod 1 is introduced into the medullary canals 2 of both bone fragments 5 and 6 and the position assumed by the shell 7 is so corrected that the shell would fall within the zone of the bone missing portion 3. Next the original limb's length is restored by complete intramedullary introduction of the central rod 1 into the fragment 5, 6 of the bone 3, the telescopic shell 7 is extended until a length is attained equal to the length of the bone missing portion 3. As a result, the edges of the shell 7 contact the ends of the fragments 5, 6 of the bone 3 and said length is fixed with the aid of one of the joints described hereinabove (FIGS 2 through 4). Thereupon the mass is dispersed and introduced through one of the holes 15 into the porous mass appearing as a bundle of fibres.

When the expanded hydrogel 8 is used as the porous mass, first the biomass is introduced into said hydrogel, whereupon both of them are introduced jointly through one of the holes 15 in the coaxial shell 7, into the space confined between the shell 7, the central rod 1, and the bone fragments 5, 6. The mixture is introduced until the biomass or its mixture with the expanded hydrogel 8 appears at the edges of both fragments 5, 6 of the bone 3. Then the wound is stitched up in layers and additional external immobilization is provided, whenever necessary.

Medicoexperimental studies of the device were carried out on experimental dogs. An annular bone fragment 30 to 35 mm long was removed from the femoral bone 3 under general anesthesia. The dia. 8 mm central rod 1 was introduced into the medullary canals 2 of both bone fragments 5 and 6 and the telescopic shell 7 was held to the rod 1, the inside diameter of the shell 7 being in excess of the diameter of the rod 1 by 5 or 6 mm.

The rod 1 had been made of a copolymer of N-vinylpyrrolidone with methylmethacrylate reinforced with a mixture of capron fibres and aromatic polyamide fibres. There were introduced into the space confined between the shell 7 and the rod 1 a bundle of capron fibres and expanded hydrogel, incorporating an additive of the bone marrow and spongy bone of the same dog. Just after surgery a large diastasis was observed between the bone fragments 5 and 6, corresponding to the length of the removed portion of the bone 3. In 141 days after surgery complete regeneration occurred and the medullary canal 2 was clearly discernible. The axis of the bone 3 was correct. The diameter of the restored bone portion was practically equal to the diameter of the original bone 3, only a hardly perceptible thickening of the bone 3 occurring on one side. Such positive results of experiments on test animals made it possible to recommend conduction of clinical trials. Given below are specific examples of practical realization of the proposed technical solution.

### Example 1

Male patient V., 42. An area of the comminuted bone 3 38 mm long, resulted from an injury to the shin bone having the medullary canal 2 11 mm in diameter. For the bone restoration procedure use was made of a set, wherein employed as the central rod 1 was a pin of a copolymer of N-vinylpyrrolidone with methylmethacrylate (the vinylpyrrolidone content being 28 to 32 molar percent) reinforced with capron fibre and incorporating an antimicrobial agent Dioxidine. The pin was shortened to get equal in length with the medullary canal 2 of the original bone 3 and was introduced retrogradedly into one of the fragments 5 of the bone 3, whereupon the coaxial telescopic shell 7 was held to the pin, said shell being made of a copolymer of N-vinylpyrrolidone with butylmethacrylate (the vinylpyrrolidone content being 48 to 56 molar percent), said copolymer containing 25 percent of a mixture of Dioxidine and Quinoxidine (1:2) and reinforced with four layers of capron fibre containing Gentamycin, the inside diameter of said shell being 16 mm, while the two portions thereof were 45 mm long. The shells 7 were provided with three supporting elements 16 to be held to the rod 1. One of the shells 7 (namely, the inner one) was provided with four pairs of the recesses 13, whereas the other (outer) shell 7 had four pairs of the fixing ridges 14. Insertion of the third pair of the ridges 14 into the third pair of the recesses 13 made it possible to bring a total length of the shell 7 to about 38 mm. Then a bundle of acid-treated capron fibre was introduced into the shell 7. Once the rod 1 had been introduced into the bone fragment 6 and the shell 7 had been installed at the place of the bone missing fragment, introduced into the bundle of fibres through one of the holes 15 was the pulp consisting of the residuals of the patient's bone marrow and the spongy component of the fragments of the lost bone 3 until the pulp appeared at the edges of the bone fragments 5, 6. Finally, the wound was stitched up in layers and a coaxial dressing was applied. Complete restoration of the fragment of a missing portion ensued in 86 days.

### Example 2

Male patient K. Missing portion of the femoral bone, 43 mm long. Use was made of the dia.14 mm central rod 1 made of a pin from a copolymer of N-vinylpyrrolydone and methylmethacrylate reinforced with modified capron fibre and of the coaxial shell 7 having a total length of its two portions 9, 10 equal up to 45 mm, said shell being made of a mixture of copolymers (taken in a ratio of 1:3) of vinylpyrrolidone with methylmethacrylate and of vinylpyrrolidone with butylmethacrylate and containing 5 percent of Gentamycin and 5 percent of orotic acid, reinforced with capron fibre. The porous mass was introduced between the rod 1 and the shell 7, said mass being based on expanded hydrogel from a copolymer of N-vinylpyrrolidone, acrylamide and ethylacrylate, comprising a biomass, i.e., homogenate of the patient's bone marrow. After stitching up the wound an external immobilization apparatus was applied. Complete restoration of the lost fragment of the bone 3 ensued in 94 days.

### Example 3

Male patient L. Missing portion of the humeral bone, 27 mm long. Use was made, as in Example 1, of the rod 1 made of the same material and having a diameter of 9 mm, and of the coaxial shell 7 composed of two portions, i. e., 9 and 10, each 25 mm long and made of a mixture of polyethylcyanacrylate and a copolymer of vinylpyrrolidone with butylmethacrylate, containing 20 percent of a mixture of Gentamycin, orotic acid, and sea-buckthorn oil (5:4:1) and reinforced with two layers of acid-treated capron fibre. A porous mass incorporated a mixture of carboxymethyl cellulose fibres and capron fibres coated with a copolymer of vinylpyrrolidone with butylmethacrylate, containing Gentamycin, sea-buckthorn oil, and homogenate of the patient's bone marrow. Complete restoration of the lost portion of the bone 3 ensued in 82 days.

### Example 4

Male patient Sh. Missing portion of the femoral bone 3, 48 mm long. Use was made, as in Example 1, of the rod 1 made of the same material and having a diameter of 12 mm, and of the coaxial shell 7 composed of two portions, i.e., 15, 16 comprising four fixing elements each and made of a mixture of copolymers of vinylpyrrolidone with methylmethacrylate and of vinylpyrrolidone with butylmethacrylate, said shell comprising a mixture of Dioxidine and Quinoxidine and being reinforced with four layers of capron fibre, containing Gentamycin. A porous mass was in the form of a bundle made up of capron fibre coated with a copolymer and incorporating sea-buckthorn oil and Gentamycin, and a biomass in the form of a pulp of the spongy bone in donor's blood. Upon stitching up of the wound a plaster-of-Paris dressing was applied. Complete restoration of the bone lost portion took 102 days.

The proposed device makes it possible to completely restore missing fragments of tubular bones, involving formation of own bone of the original dimensions.

A total treatment course with the use of a known device ranges between 300 and 360 days, whereas that with the use of the proposed device lasts from 90 to 100 days. No complications after application of the proposed device occurred.

### Applicability

The invention can find most utility when applied for restoration of bone missing portions due to total defects of long tubular bones following extensive injuries or wounds, or after vast pathological changes of the bone tissue (e.g., tumors or tuberculous affections).

## Claims

1. A device for restoration of tubular bone missing portion, comprising a central rod (1) insertable into a medullary canal (2) of fragments (5, 6) of a bone (3) and a tubular element (4) made from a synthetic material and adapted to be fitted on the rod (1) at the place of the bone missing portion and being equidimensional with the missing portion of the bone (3), characterized in that the tubular element (4) is shaped as a shell (7) from polymers biocompatible with the bone tissue, and a layer (8) of a porous polymer material, insertable in between the rod (1) and the shell (7) and incorporating a biomass of homogenized bone tissues.

2. A device as claimed in Claim 1, characterized in that the shell (7) is shaped as a telescopic structure composed of at least two tubular elements (9, 10).

3. A device as claimed in Claim 1, characterized in that the layer (8) of a porous polymer material is shaped as a bundle of fibres from biocompatible polymers.

4. A device as claimed in Claim 1, characterized in that the layer (8) of a porous polymer material is made from expanded hydrogel.

## Patentansprüche

1. Gerät zur Wiederherstellung eines verlorenen Teiles eines Röhrenknochens, enthaltend einen zentralen Stab (1), der in einen Markkanal (2) von Fragmenten (5, 6) eines Knochens (3) einfügbar ist, und ein rohrförmiges Element (4), das aus einem synthetischen Werkstoff hergestellt ist und angepaßt ist, um an dem Stab (1) an der Stelle des verlorenen Teils des Knochens eingefügt zu werden und das die gleichen Abmessungen wie der verlorene Teil des Knochens (3) aufweist, dadurch gekennzeichnet, daß das rohrförmige Element (4) gestaltet ist als eine Ummantelung (7) aus Polymeren, die mit dem Knochengewebe biokompatibel sind, und eine Schicht (8) aus einem porösen Polymerwerkstoff, die zwischen den Stab (1) und die Ummantelung (7) einfügbar ist und eine Biomasse aus homogenisierten Knochengeweben inkorporiert.

2. Gerät nach Anspruch 1, dadurch gekennzeichnet, daß die Ummantelung (7) als eine Teleskopstruktur gebildet ist, die aus wenigstens zwei rohrförmigen Elementen (9, 10) zusammengesetzt ist.

3. Gerät nach Anspruch 1, dadurch gekennzeichnet, daß die Schicht (8) aus einem porösen Polymerwerkstoff als ein Faserbündel aus biokompatiblen Polymeren gebildet ist.

4. Gerät nach Anspruch 1, dadurch gekennzeichnet, daß die Schicht (8) aus einem porösen Polymerwerkstoff aus expandiertem Hydrogel hergestellt ist.

## Revendications

1. Dispositif de restauration de la partie manquante d'un os tubulaire, comprenant une tige centrale (1) destinée a être introduite dans un canal médullaire (2) de fragments (5, 6) d'un os (3) et un élément tubulaire (4) formé d'un matériau de synthèse et destiné à être placé sur la tige (1) à l'emplacement de la partie osseuse manquante et ayant des dimensions égales à celles de la partie osseuse manquante (3), caractérisé en ce que l'élément tubulaire (4) a la configuration d'une enveloppe (7) formée de polymères biocompatibles avec le tissu osseux, et une couche (8) d'un matériau polymère poreux, destinée à être introduite entre la tige (1) et l'enveloppe (7) et contenant une biomasse de tissus osseux homogénéisés.

2. Dispositif selon la revendication 1, caractérisé en ce que l'enveloppe (7) a la forme d'une structure télescopique composée d'au moins deux éléments tubulaires (9, 10).

3. Dispositif selon la revendication 1, caractérisé en ce que la couche (8) du matériau polymère poreux a la configuration d'un faisceau de fibres de polymères biocompatibles.

4. Dispositif selon la revendication 1, caractérisé en ce que la couche (8) du matériau polymère poreux est formée d'un hydrogel expansé.
